# EUROPÄISCHE PATENTSCHRIFT

(11) **EP 2 515 960 B1**
(45) Veröffentlichungstag und Bekanntmachung des Hinweises auf die Patenterteilung: **19.03.2014**
(21) Anmeldenummer: 10812844.8
(22) Anmeldetag: 22.12.2010
(51) Int. Cl.: A61L 31/00, A61F 2/36

(54) **IMPLANTAT, VERFAHREN ZUM HERSTELLEN EINES SOLCHEN IMPLANTATS SOWIE VERWENDUNG**
IMPLANT, METHOD FOR PRODUCING SUCH AN IMPLANT, AND USE
IMPLANT, PROCÉDÉ DE PRODUCTION ET UTILISATION D'UN TEL IMPLANT

(30) Priorität: 23.12.2009 DE 102009060546
(43) Veröffentlichungstag der Anmeldung: 31.10.2012
(73) Patentinhaber: Jockenhövel, Stefan, 52072 Aachen (DE)
(72) Erfinder: DEICHMANN, Thorsten, 23556 Lübeck (DE); GRIES, Thomas, 52072 Aachen (DE); SCHMITZ-RODE, Thomas, 52070 Aachen (DE); MAHNKEN, Andreas, 52070 Aachen (DE); WEINANDY, Stefan, 52070 Aachen (DE)
(74) Vertreter: Castell, Klaus
(86) Internationale Anmeldenummer: PCT/DE2010/001506
(87) Internationale Veröffentlichungsnummer: WO 2011/076186

(56) Entgegenhaltungen:
- WO-A1-01/87192
- US-A1- 2003 138 950
- YASUHIDE NAKAYAMA ET AL: "Development of in vivo tissue-engineered autologous tissue-covered stents (biocovered stents)", JOURNAL OF ARTIFICIAL ORGANS ; THE OFFICIAL JOURNAL OF THE JAPANESE SOCIETY FOR ARTIFICIAL ORGANS, SPRINGER-VERLAG, TO, Bd. 10, Nr. 3, 20. September 2007 (2007-09-20), Seiten 171-176, XP019520356, ISSN: 1619-0904, DOI: 10.1007/S10047-007-0376-1
- TOSHIHIKO SHIROTA ET AL: "Intralumenal Tissue-Engineered Therapeutic Stent Using Endothelial Progenitor Cell-Inoculated Hybrid Tissue and in Vitro Performance", TISSUE ENGINEERING, Bd. 9, Nr. 3, 1. Juni 2003 (2003-06-01), Seiten 473-485, XP55002877, ISSN: 1076-3279, DOI: 10.1089/107632703322066651
- H.J. PATEL ET AL: "A Combined Strategy To Reduce Restenosis for Vascular Tissue Engineering Applications", BIOTECHNOLOGY PROGRESS, Bd. 22, Nr. 1, 3. Februar 2006 (2006-02-03), Seiten 38-44, XP55002881, ISSN: 8756-7938, DOI: 10.1021/bp050135e
- SHIROTA T ET AL: "Fabrication of endothelial progenitor cell (EPC)-seeded intravascular stent devices and in vitro endothelialization on hybrid vascular tissue", BIOMATERIALS, ELSEVIER SCIENCE PUBLISHERS BV., BARKING, GB, Bd. 24, Nr. 13, 1. Juni 2003 (2003-06-01), Seiten 2295-2302, XP004420020, ISSN: 0142-9612, DOI: 10.1016/S0142-9612(03)00042-5

## Beschreibung

Die Erfindung betrifft ein Implantat, ein Verfahren zum Herstellen eines solchen Implantats sowie die Verwendung eines solchen Implantats.

Röhrenförmige Organe und Strukturen wie Blutgefäße, die Speiseröhre, Därme, die ableitenden Gänge endokriner Düsen und die Harnröhre können sämtlich von Stenosen betroffen sein, also einer Verengung oder einem Verschluss des Lumens. Strikturen können insbesondere von traumatischen oder organischen Erkrankungen verursacht werden. Die Symptome können von einer leichten Reizung und Unbehagen bis zur Lähmung und Tod reichen.

Der wissenschaftliche Hintergrund der Erfindung wird nachstehend anhand der perkutanen Stentangioplastie als ein sehr gut verständliches Beispiel für eine Stentapplikation beschrieben, ist jedoch ohne Weiteres auf andere Stentapplikationen im nicht kardiovaskulären Bereich oder auf verwandte Applikationen übertragbar, wie beispielsweise einen Okkluder, eine stentgestütze Klappe oder eine flußregulierende Struktur mit entsprechender Anpassung der funktionellen Zellen.

Die perkutane Stentangioplastie peripherer und koronarer Gefäße hat sich inzwischen als klinisches Routineverfahren etabliert.

Dennoch sind die Offenheitsraten mit abnehmendem Gefäßkaliber unbefriedigend, insbesondere in der femoralen oder femero-poplitealen Strombahn. Verschlussraten von über 20% nach sechs Monaten werden beobachtet.

Die konventionelle Stenttherapie hat die Zielsetzung, atherosklerotischen Plaque mittels eines Ballonkatheters aufzudehnen und mit Hilfe eines gleichzeitig oder anschließend eingebrachten Stents an der Gefäßwandung zu stabilisieren.

Ein wesentliches Problem dieser Technik wird als In-Stent-Restenose bezeichnet. Hierbei kommt es zu einem erneuten Verschluss im Bereich der Stentstruktur. Aufgrund der Ballondilatation sowie der eingebrachten Stentstruktur kommt es zur einer Fremdkörperreaktion, welche wiederum zu einer Zellproliferation und einer Zelleinsprossung durch die offene Porenstruktur des Stents führt. Diese schnelle Gewebevermehrung führt zu einer Gefahr, denn sowohl das Fremdmaterial als auch der atherosklerotische Plaque selbst sind thrombogen und können schnell zu einer Thrombusbildung bis hin zu einem kompletten Verschluss des Gefäßes führen.

In Analogie zu den kardiovaskulären Stentprothesen kommt es in anderen Bereichen, wie beispielsweise der Urologie (Harnröhre, Harnleiter oder Nierenbecken) der Hals-Nasen-Ohren-Gegend (Nasennebenhöhlenöffnung, Trachealstents, Bronchialstents), der Augenheilkunde (Kammerwasserabfluss) und der Chirurgie (Oesophagus, Gallen- und Pankreasgänge, Darmanastomosen, Stenteinbringung in Tumorgebiete usw.) zu vergleichbaren Problematiken.

Ebenso sind Stents als Fügetechnik zwischen tubulären Strukturen oder zur Verbindung von biologischen mit künstlichen Systemen von großem Interesse. Generell ist ein "Stent" einfach als eine "Gefäßstütze" zu verstehen.

Auch die Regulation eines Durchflusses kann in ihrer Funktion optimiert werden, zum Beispiel bei einem Implantat mit einer integrierten Klappenfunktion und/oder für die Regulierung des Abflusses oder Zuflusses, zum Beispiel des Liquor cerebri beim Hydrocephalus, des Gallenflusses bei Tumoren im Bereich des Pankreas oder des Kammerwassers beim Glaukom.

Ein weiteres Anwendungsgebiet ist der Verschluss von angeborenen oder erworbenen Perforationen oder andersartigen Öffnungen wie beispielsweise der Verschluss von iatrogenen Darmperforationen, der Verschluss von Fisteln oder der Verschluss von Vorhof- oder Ventrikelseptumdefekten.

Die DE 601 15 235 T2 beschreibt einen aus Gewebe aufgebauten Stent zur Behandlung von Verengungen der Harnröhre oder generell zur Verwendung als Gefäßstent. Es werden biodegradierbare Polymere verwendet, welche kurz als biologische Polymere bezeichnet werden. Diese schaffen eine tubuläre Struktur, welche anschließend mit Knorpelzellen (Chondrozyten) besiedelt werden. Ziel der chondrozytären Besiedelung ist es, eine elastische, vollständig autologe Stentstruktur zu schaffen.

Die US 2003/0138950 A1 beschreibt die Nutzung von zellären Gewebeplatten, welche um Stentstrukturen gewickelt werden, um einen Stent mit vitalem Gewebe zu beschichten.

Beide vorstehend genannten Patentveröffentlichungen seien hinsichtlich ihres Gesamtoffenbarungsgehalts im Wege der Referenzierung hier ebenfalls als offenbart zu verstehen.

Nakayama Y et al. publizierten 2001 im Journal of Artificial Organs (DOI 10.1007/s10047-007-0376-1) die Entwicklung eines in vivo tissue engineerten, also gewebegezüchteten, autologen gewebeüberzogenen Stents. Hierbei wurde eine konventionelle Stentstruktur über einem zentralen Silikonkern fixiert und subkutan implantiert. Aufgrund der Reaktion des umgebenden Gewebes auf das Fremdmaterial kam es zu einer Abkapselung des Stents durch eine fibröse Ummantelung.

Der Erfindung liegt die Aufgabe zugrunde, ein verbessertes Implantat zur Verfügung zu stellen.

Diese Aufgabe löst ein Implantat mit einem mechanischen Komponente und einer biologisch aktiven Komponente, wobei die mechanische Komponente eine formgebende Stützstruktur aufweist und die biologisch aktive Komponente ein die Stützstruktur besiedelndes bioverträgliches Material mit Zellen aufweist, wobei die Zellen ein vitales, vaskulär aktives Gewebe bilden und die Stützstruktur vollständig in dieses aktive Gewebe integriert ist.

Das Konzept eines solchen Implantats wirkt somit - am Beispiel des Stents mit dem Problem einer Re-Instent-Stenose - den Hauptursachen für eine Problematisierung des Implantats nach dem Einbringen entgegen:

Das Prinzip des vorgeschlagenen Implantats beruht auf der vollständigen Integration der Stützstruktur in ein bioverträgliches Material mit Zellen, sodass kein direkter Kontakt zwischen der Stützstruktur und der Blutphase zustande kommt.

Bevorzugt besteht das Implantat ausschließlich aus der mechanischen Komponente und der biologisch aktiven Komponente.

In einer vorteilhaften Ausführungsform für bestimmte Anwendungszwecke ist das Implantat als Stent ausgebildet, mit einer tubulären Stützstruktur, welche eine Radialsteifigkeit für eine Stützwirkung eines Gefäßes aufweist.

Bei der Anwendung der Erfindung auf einen Stent führt die Biologisierung der gesamten Stentstruktur - und nicht nur wie bisher bekannt der Stentstreben - zu einem entscheidenden Schritt für eine lange Offenheitsrate.

Die Erfindung grenzt sich von den bisherigen Ansätzen sogenannter drug-eluting Stents ab, also Wirkmittel-eluierender Stents. Bei den drug-eluting Stents soll über die Freisetzung von Zytostatika eine Zelleinsprossung verhindert werden. Es hat sich jedoch gezeigt, dass neben der Reduktion der Zelleinsprossung auch die Neubesiedelung verhindert wird. Insbesondere betrifft dies auch die Neubesiedelung mit Endothelzellen, welche allerdings für eine physiologische Biokompatibilität und Hämokompatibilität essentiell sind.

Das vorgeschlagene Implantat soll bevorzugt räumlich eigensteif sein, wobei die Steifigkeit in unterschiedlichen Raumrichtungen unterschiedlich stark ausgeprägt sein kann.

Die Eigensteifigkeit soll im Wesentlichen durch die "Stützstruktur" hervorgebracht werden.

Für die Stützstruktur können vor allem Metalle, Polymere oder Kombinationen aus verschiedenen Werkstoffen zum Einsatz kommen. Es können Werkstoffe oder Bauteile mit oder ohne Strukturgedächtnis (shape memory effect) zum Einsatz kommen.

Die Stützstruktur kann insbesondere eine textiltechnische Zusammensetzung aufweisen, vor allem kann sie als Gewirk, Geflecht, Gewebe, Gestrick oder als Vlies vorliegen. Alternativ oder kumulativ kann ein Schnittverfahren, ein Laserbearbeitungsverfahren und/oder Abformungsverfahren zum Einsatz kommen.

Vom Ansatz nach Nakayama Y et al. grenzt sich das hier vorgeschlagene Implantat dadurch ab, dass bei Nakayama lediglich eine Beschichtung des Stents mit minderwertigem Bindegewebe in vivo stattfindett. Das hier vorgeschlagene Konzept ermöglicht im Gegensatz dazu die Herstellung eines funktionell aktiven Gewebes in vitro, also mittels eines tissue engineering-Verfahrens (Gewebezüchtungsverfahrens).

Von der DE 601 15 235 T2 grenzt sich das hier vorgeschlagene Verfahren ebenfalls ab: im Gegensatz zum hier vorgeschlagenen Verfahren wird in der DE 601 15 235 T2 kein vaskulär aktives Gewebe erzeugt. Insbesondere wird keine funktionelle Endothelzellfläche erzeugt.

Von der US 2003/0138950 A1 grenzt sich das hier vorgeschlagene Implantat schon dadurch ab, dass hier eine vollständige Integration der Stentstruktur in lebendiges, biologisch-aktives Gewebe erfolgt. Dadurch kann ein vollständiges Beschichten erzielt werden.

Außerdem grenzt sich der hiesige Vorschlag dadurch ab, dass die zu applizierende Zellsorte gezielt ausgewählt und für den geplanten Einsatzort optimiert wird.

Es wird vorgeschlagen, dass der Stent radial komprimierbar und/oder expandierbar gestaltet ist, insbesondere ballonexpandierbar oder selbstexpandierbar. Es versteht sich, dass die biologisch-aktive Komponente so elastisch oder plastisch gestaltet sein sollte, dass die vollständige Integration der Stützstruktur in das aktive Gewebe beim Komprimieren und/oder Expandieren des Stents erhalten bleibt.

Eine bevorzugte Ausführungsform eines Stents, welcher gemäß der hier vorliegenden Erfindung gefertigt wurde, lässt das aktive Gewebe einen Hohlzylinder formen, wobei die tubuläre Stützstruktur des Stents in die Zylinderwand des aktiven Gewebes integriert ist.

Als "aktiv" oder "funktionell aktiv" werden im Rahmen der hier vorliegenden Offenbarung lebendige Zellen bezeichnet. Diese können durch die Produktion und Freisetzung von Faktoren mit oder ohne vorherige Stimulation oder durch ihre physiologische Lokalisation zum Erhalt der Implantatfunktion beitragen.

Bei der Herstellung des vitalen Implantats, insbesondere des vitalen Stents, aus einer selbstexpandierenden Stützstruktur und einem vitalen, tissue engineerten Gefäßkonstrukt ist deshalb darauf zu achten, dass lebendige Zellen für das Gefäßkonstrukt zum Einsatz kommen.

Ein Stent ist bevorzugt an der tubulären Stützstruktur dichtend ausgebildet, sodass ein geschädigter Wandabschnitt eines Gefäßes, insbesondere ein atherosklerotischer Wandabschnitt, nach Implantation des Stents vollständig aus einer Blutstrombahn ausgeschlossen ist. Wenn die biologisierte Stentstruktur den atherosklerotischen Wandabschnitt vollständig aus der Blutstrombahn ausschließt, wird die Gefahr einer Re-Instent-Stenose erheblich herabgesetzt.

Besonders bevorzugt ist das Implantat, wenn das aktive Gewebe eine Endothelzellfunktionsfläche aufweist, insbesondere ein Endothelzellfläche mit funktionell aktiven Endothelzellen. Eine solche Schicht sorgt für eine hohe Hämokompatibilität.

Die lebendige, funktionell aktive Endothelzellschicht oder eine vergleichbare Schicht mit ähnlicher Funktion ist bevorzugt im Neolumen aufgebracht. Das Konzept des hier vorgeschlagenen Stents wirkt dann gleich allen Hauptursachen der Re-Instent-Stenose entgegen.

Die Endothelzellfunktionsfläche ist bevorzugt als luminale Besiedelung vorgesehen, also zu demjenigen Bereich hin gewandt, in welchem durch das Gefäß eine biologische Flüssigkeit nach dem Einsatz des Implantats strömen soll.

Es sei ausdrücklich darauf hingewiesen, dass sich dieses Prinzip mit entsprechend abgeänderten, lokal jeweils relevanten Zellen, beispielsweise Urothelzellen, Zellen der Mundschleimhaut bzw. des Magen- Darm-Traktes auf andere Stenttypen aus anderen klinisch und biologisch relevanten Bereichen übertragen lässt. Auch Okkludersysteme zum Verschluss von angeborenen oder erworbenen Defekten, beispielsweise von Vorhof- oder Ventrikelseptumdefekten, können auf diese Weise biologisiert werden.

Dieser Aspekt beinhaltet eine noch deutlichere Verbesserung des Stands der Technik. So wurden bislang sogenannte "cell capture stents" vorgeschlagen, welche erst nach der Implantation Zellen (z.B. Endothelzellen oder endotheliale Progenitorzellen) an der Oberfläche binden, während das hier vorgeschlagene Implantat diese Zellen zum Zeitpunkt der Implantation direkt und gezielt zur Verfügung stellt, also mit optimaler Zellbeschichtung entsprechend der angestrebten Funktion, beispielsweise mit Endothelzellen für eine cardiovasculäre Applikation, mit respiratorischem Epithel für eine endobronchiale Applikation, mit Urothel für eine Harnleiter/Harnröhren-Applikation etc.

Die Stützstruktur des Implantats kann scheibenförmig oder zylindrisch geschlossen ausgebildet sein, sodass das Implantat als Verschluss für einen Vorhof- oder Ventrikelseptumdefekt oder für einen offenen ductus botalli ohne Weiteres einsetzbar ist.

Die Stützstruktur des Implantats kann eine Verankerung zu einer größeren, externen artifiziellen Struktur aufweisen oder zu dieser bereits verankert sein.

Beispielsweise sei an eine Verankerung zu einer Polyurethanstruktur gedacht. Weitere Möglichkeiten liegen in einer Verankerung zu einer Kunstharnblase, zu einem Kunstherz oder zu einem Medikamentenapplikator, wobei diese Aufzählung nicht als vollständig zu verstehen ist. Entscheidend ist nur die Verbindung (Anastomosierung) von artifiziellen Strukturen an anatomische tubuläre Strukturen.

Die Verankerung in der externen Struktur kann insbesondere während des Formprozesses der Stützstruktur erfolgen.

Hinsichtlich der zeitlichen Reihenfolge beim Herstellen einer solchen Verankerung und Verbindung sei insbesondere daran gedacht, ein Ankerelement oder mehrere Ankerelemente zunächst mit der Stützstruktur zu verbinden und anschließend mit der externen Struktur zu verankern oder das beziehungsweise die Ankerelemente zunächst an der externen Struktur zu verankern und anschließend mit der Stützstruktur zu verbinden. Selbstverständlich ist es auch möglich, Ankerelemente gleichzeitig mit der externen Struktur zu verankern und mit der Stützstruktur zu verbinden.

Hinsichtlich der Verbindung zwischen den Ankerelementen und der Stützstruktur sei insbesondere daran gedacht, dass die Ankerelemente hervorstehende Konstruktionselemente der Stützstruktur sein können. Alternativ ist daran zu denken, ein Ankerelement strukturell in die Stützstruktur zu integrieren, beispielsweise durch Einweben oder Einwirken. Je nach technischem Aufbau der Stützstruktur bieten sich unterschiedlichen Verbindungsweisen zwischen der Stützstruktur und den Ankerelementen an.

Insbesondere wenn das Implantat für eine künstliche Blase und ein künstlichen Blasenausgang gedacht ist, aber nicht auf diesen Fall eingeschränkt, wird vorgeschlagen, dass die Stützstruktur von der externen Struktur fort verläuft, insbesondere in einem rechten Winkel auf einer Oberfläche der externen Struktur stehend.

Es wurde bereits erwähnt, dass das aktive Gewebe bevorzugt mittels eines Gefäßzüchtung-Verfahrens hergestellt ist. Ein solches Verfahren wird üblicherweise als tissue-engineering-Verfahren bezeichnet.

Es wird vorgeschlagen, dass das aktive Gewebe vaskuläre Muskelzellen und Endothelzellen aufweist.

Unabhängig davon, ob diese oder andere Zellarten zum Einsatz kommen, ist es von Vorteil, wenn das aktive Gewebe mehrere Schichten unterschiedlicher Zellzusammensetzung aufweist. Vor allem sei daran gedacht, eine Außenschicht und eine Innenschicht sowie unter Umständen eine oder mehrere Mittelschichten am Implantat vorzusehen. Wenn das Implantat als Stent ausgebildet ist, wird vorgeschlagen, dass die einzelnen Schichten des aktiven Gewebes einzeln oder allesamt eine Zylinderform aufweisen.

Die Kombination unterschiedlicher Zellen kann nicht nur schichtweise erfolgen, sondern auch durch Vermischung unterschiedlicher Zellen innerhalb einer Schicht.

Ohne Weiteres können sowohl menschliche als auch tierische Zelltypen verwendet sein.

Bevorzugt ist die Besiedelung mit dem aktiven Gewebe mittels eines Spritzguss- oder anderen Gussverfahrens erzeugt. Alternativ sei an ein dip-coating-Verfahren, ein Sprühverfahren oder ein LIFT-Verfahren gedacht, also an ein laser induced forward transfer-Verfahren.

An der Stützstruktur ist bevorzugt für die Besiedelung mit dem aktiven Gewebe eine Trägerstruktur vorgesehen, insbesondere ein Hydrogel oder eine synthetische Trägerstruktur. Hydrogele können beispielsweise fibrinbasiert sein, aber auch auf der Basis anderer biologischer Hydrogele aus beispielsweise extrazellulärer Matrix oder Einzelkomponenten der extrazellulären Matrix, wie Kollagen, Elastin, Proteoglykane, Glykoproteine oder tierischen Ursprungs wie Chitosan, wobei diese Aufzählung nicht abschließend ist. Synthetische Trägerstrukturen können auch beispielsweise aus PEG sein.

Eine besonders biokompatible Reaktion des Implantats wird dann erwartet, wenn die Zellbesiedlung der Stützstruktur als lückelose und vor allem dünne Beschichtung mit einer Dicke mit Mittelwerten zwischen 5 µm und 100 µm mm erzeugt ist, insbesondere zwischen 5 µm und etwa 50 µm, vor allem in der Gesamtbeschichtung auf der Stützstruktur etwa 10 µm bis 20 µm.

Die eigentliche Zellbeschichtung wird bevorzugt dem physiologischen Aufbau entsprechen.

Bei der vaskulären Applikation bedeutet dies idealerweise einen Monozelllayer von etwa 2 µm bis etwa 10 µm, vor allem etwa 5 µm, während das Urothel bevorzugt mehrschichtig ist und etwa 30 µm bis 70 µm Schichtdicke aufweisen kann, vor allem etwa 50 µm Schichtdicke.

In konkreter Ausgestaltung wird vorgeschlagen, dass das Implantat als eines der im Anspruch 19 genannten Implantatformen hergestellt ist.

Hinsichtlich eines Herstellverfahrens eines Implantats wie vorstehend beschrieben wird vorgeschlagen, dass das aktive Gewebe mittels eines tissue-engineering-Verfahrens erstellt wird. Dies wurde bereits erläutert.

Hinsichtlich des Verfahrens zum Besiedeln der Stützstruktur wird vor allem ein Gussverfahren vorgeschlagen, insbesondere ein Spritzgussverfahren. Nach den bisherigen Überlegungen der Erfinder eignet sich vor allem ein Spritzgussverfahren besonders zum Herstellen einer vollständigen, dünnen Beschichtung der Stützstruktur.

Auch ein Sprühverfahren oder ein Tauchverfahren scheint sehr geeignet.

Schließlich wird vorgeschlagen, das Implantat zusätzlich zu den vorstehend genannten Eigenschaften als ein mehrphasiges drug-release-System zu gestalten, insbesondere mit einer kovalenten Ankoppelung, mit inkorporierten Sphären, mit eingegossenen Substanzen wie Medikamenten und Wachstumsfaktoren oder ähnlichem.

Die Erfindung wird nachstehend anhand eines Ausführungsbeispiels unter Bezugnahme auf die Zeichnungen näher erläutert. Dort zeigen
- Figur 1: schematisch in einem Längsschnitt ein geschädigtes Blutgefäß mit einem eingesetzten Stent,
- Figur 2: in einer fotografischen Teildraufsicht eine Stentstruktur, welche vollständig in ein vitales Gewebe eingebettet ist und
- Figur 3: schematisch einen Aufbau eines endobronchialen Stents.

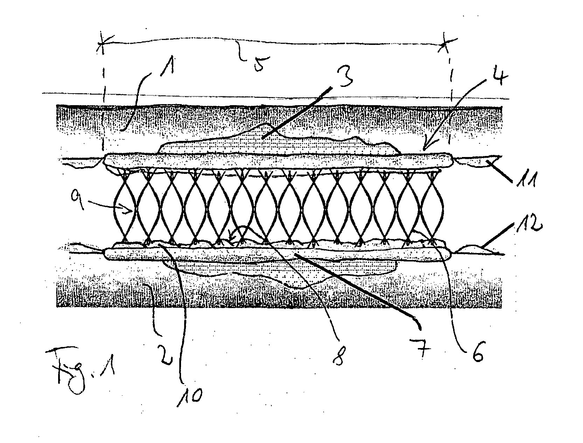

Das Blutgefäß 1 in Figur 1 besteht aus einer im Wesentlichen zylindrischen, schlauchförmigen Grundgestalt 2. In Folge athesklerotischen Plaques 3 war das Blutgefäß 1 verschlossen.

Bei der perkutanen Aufweitung des Blutgefäßes 1 wurde ein Stent 4 an die geschädigte Stelle eingebracht. Der Stent 4 hat eine Länge 5, welche mindestens die geschädigte Stelle im Blutgefäß 1 abdeckt.

Der Stent 4 besteht im Wesentlichen aus einer selbstexpandierenden, metallenen Stützstruktur 6 (in der Ansicht zwischen den zwei geschnittenen Bereichen ohne Einbettung in Gewebe gezeigt) und einem zylindrischen Gewebe 7 mit lebendigen Zellen.

Die Stützstruktur 6 ist vollständig in das zylindrische Gewebe 7 eingebettet. Das zylindrische Gewebe 7 besiedelt somit vollflächig und lückenlos die Stützstruktur 6.

An einer Innenseite 8 des zylindrischen Gewebes 7, also zu einem Lumen 9 hin gerichtet, befindet sich eine Schicht 10, welche aus einer Besiedelung mit Endothelzellen besteht.

Die Schicht 10 aus Endothelzellen, jedenfalls aber das zylindrische Gewebe 7, reichen bis möglichst nah an natives Endothel 11, 12 heran.

Der Stent 4 besteht somit im Wesentlichen aus einem nativ eingebetteten Träger mit einer Endothel-Beschichtung. Das Gewebe ist lebendig und weist eine differenzierte Zellzusammensetzung auf.

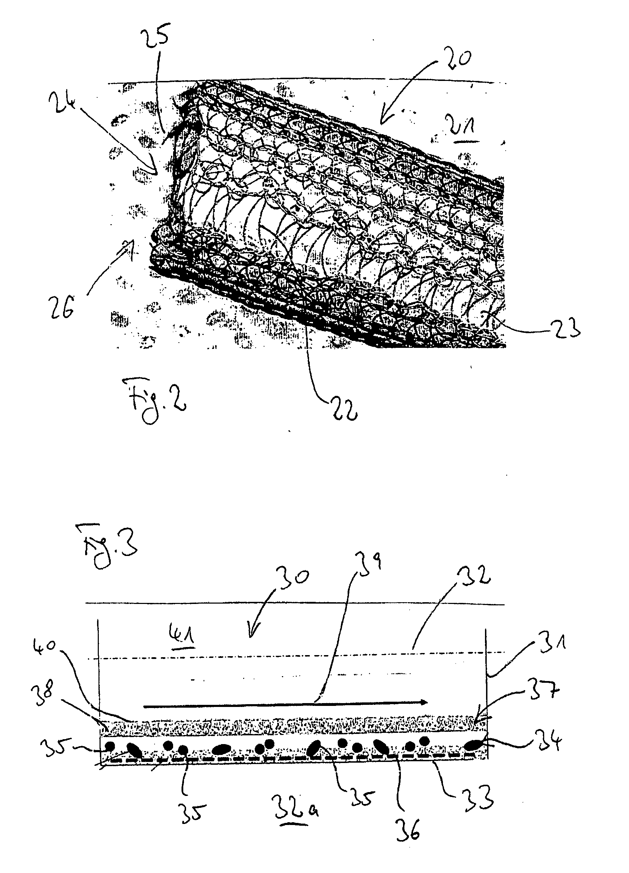

Der fotografisch dargestellte Stent 20 in Figur 2 ist ein Labormuster, welches dem Stent aus Figur 1 prinzipiell im Aufbau entspricht. Der Stent 20 liegt auf einem strukturierten Untergrund 21 zum Zwecke der Fotografie auf.

Eine Stützstruktur 22 des Stents 20 besteht aus PLA, welches in langen Fäden vorlag, welche miteinander verwirkt worden sind. Hierbei haben sie eine zylindrische Grundstruktur für den Stent 20 erhalten.

Die Stützstruktur 22 ist vollständig in zellulär aktives, lebendiges Fibringel 23 (nahezu farblos) eingebettet. Radial ragt die Stützstruktur weder nach außen noch nach innen zum Lumen 24 aus dem Fibringel 23 hervor. Axial steht das PLA mit einigen Fädenenden 25 (exemplarisch gekennzeichnet) aus dem Fibringel 23 hervor, da das Labormuster an einer Schnittstelle 26 einfach durchgeschnitten wurde.

Der Aufbau 30 für einen endobronchialen Stent 31 in Figur 3 ist ebenfalls zylindrisch um eine Achse 32 herum aufgesetzt.

Um ein endobronchiales Gefäß nach einer Lungenkrebsoperation vor einer Restenose zu schützen, wird der Stent 31 vorgeschlagen.

Zu einer Außenseite 32a hin ist der Stent 31 mit einer Trennlage in Form einer elastischen Folie 33 umgrenzt. Für die Trennlage können unterschiedliche Arten von Kunststoffen eingesetzt werden. Insbesondere sei an Polymere, an Pulyurethan und/oder an Polycarbon-Urethan gedacht. Es lässt sich auch darüber nachdenken, ein Sprüh- und/oder Schwammverfahren zum Einsatz zu bringen.

Innerhalb der PU-Folie 33 befinden sich mehrere funktionale Schichten:

Die mechanische Basis bildet eine selbstexpandierende gestrickte Stützstruktur 34 aus Nitinol-Fäden 35 (exemplarisch gekennzeichnet).

Direkt bei der Stützstruktur 34 , somit radial außen im Stent 31, sind Mikrosphären oder Nanosphären 36 (exemplarisch gekennzeichnet) angeordnet. Diese beinhalten tumorspezifische Wirkmittel. Konkret können auf diese Weise Kügelchen mit Wirkmittel mit niedrigem Molekulargewicht und Antikörpern eingebettelt sein. Die Mittel und Antikörper sind gezielt dergestalt ausgewählt, dass sie ein Tumorwachstum an der Außenseite des Stents unterdrücken, gleichzeitig soll die Poliferation und Funktion einer inneren Schicht 37 mit Atemwegsepithelzellen nicht negativ beeinflusst werden.

Zusätzlich können auf Wunsch kortikosteroide Wirkmittel in die Kügelchen eingebracht sein, um etwaigen akuten Entzündungsreaktionen nach Einbringen des Stents 31 zu begegnen.

Die innere Schicht 37 des Stents 31 basiert auf einer Trägerlage 38 aus Fibringel, besetzt mit lebendigen, funktionell aktiven Atemwegsepithelzellen. Hierdurch wird eine mukoziliäre Klärung (angedeutet durch einen Pfeil 39 in Klärungsrichtung) auf Dauer sichergestellt.

Die Epithelzellen, ausgestattet mit einer Flimmerhaaroberfläche 40, können der Nasenschleimhaut entnommen sein.

Die Isolierung und Kultivierung der Epithelzellen ist ohne Weiteres über einen Zeitraum von zwei Jahren möglich.

Somit ist der Stent 31 in sich stabil, gewährt nach innen zum Lumen 41 eine sichere Funktion, therapiert lokal den Tumor durch die mikroporöse Trennlage nach außen, bietet eine mechanische Trennung über ebenjene Trennlage, wobei gleichzeitig die Trennlage für eine sichere Verankerung des Stents 31 in das umgebende Gewebe 42 sorgt.

### Bezugszeichenliste

- 1: Blutgefäß
- 2: Gefäßstruktur
- 3: Plaque
- 4: Stent
- 5: Länge
- 6: Stützstruktur
- 7: zylindrisches Gewebe
- 8: Innenseite
- 9: Lumen
- 10: Schicht
- 11: natives Endothel
- 12: natives Endothel
- 20: Stent
- 21: Untergrund
- 22: Stützstruktur
- 23: Fibringel
- 24: Lumen
- 25: Fädenenden
- 26: Schnittstelle
- 30: Aufbau
- 31: endobronchialer Stent
- 32: Achse
- 32a: Außenseite
- 33: PU-Folie
- 34: Stützstruktur
- 35: Nitinol-Fäden
- 36: Sphären
- 37: innere Schicht
- 38: Trägerlage
- 39: Pfeil
- 40: Flimmerhärchenoberfläche
- 41: Lumen
- 42: umgebendes Gewebe

## Patentansprüche

1. Implantat mit einer mechanischen Komponente und einer biologisch aktiven Komponente, wobei die mechanische Komponente eine formgebende Stützstruktur (6, 22, 34) aufweist und die biologisch aktive Komponente ein die Stützstruktur (6, 22, 34) besiedelndes bioverträgliches Material mit Zellen aufweist, ***dadurch gekennzeichnet, dass*** die Zellen ein vitales, vaskulär aktives Gewebe (7) bilden und die Stützstruktur (6, 22, 34) vollständig in das aktive Gewebe (7) integriert ist,
wobei das Implantat als Stent (4, 20, 31) ausgebildet ist, mit einer tubulären Stützstruktur (6, 22, 34), welche eine Radialsteifigkeit für eine Stützwirkung eines Gefäßes (1) aufweist,
wobei das aktive Gewebe (7) einen Hohlzylinder formt und die tubuläre Stützstruktur (6, 22, 34) in eine Zylinderwand integriert ist,
wobei eine vollständige Integration der tubulären Stützstruktur (6, 22, 34) in lebendiges, biologisch-aktives Gewebe vorliegt, wodurch ein vollständiges Beschichten erzielt werden kann, und
wobei das als "aktiv" bezeichnete Gewebe lebendige Zellen umfasst, welche durch die Produktion und Freisetzung von Faktoren mit oder ohne vorherige Stimulation oder durch ihre physiologische Lokalisation zum Erhalt der Implantatfunktion beitragen können.

2. Implantat nach Anspruch 1, ***dadurch gekennzeichnet, dass*** es ausschließlich aus den beiden Komponenten besteht.

3. Implantat nach Anspruch 1 oder 2, ***dadurch gekennzeichnet, dass*** der Stent (4, 20. 31) radial komprimierbar und/oder expandierbar gestaltet ist, insbesondere ballonexpandierbar oder selbstexpandierbar.

4. Implantat nach einem der vorstehenden Ansprüche, ***dadurch gekennzeichnet, dass*** der Stent (4, 20. 31) an der tubulären Stützstruktur (6, 22, 34) dichtend ausgebildet ist, sodass ein geschädigter Wandabschnitt eines Gefäßes (1), insbesondere ein artherosklerotischer Wandabschnitt, nach Implantation des Stents (4, 20. 31) vollständig aus einer Blutstrombahn ausgeschlossen ist.

5. Implantat nach einem der vorstehenden Ansprüche, ***dadurch gekennzeichnet, dass*** das aktive Gewebe (7) eine Endothelzellfunktionsfläche aufweist, insbesondere eine Endothelzellfläche mit funktionell aktiven Endothelzellen.

6. Implantat nach Anspruch 5, ***dadurch gekennzeichnet, dass*** die Endothelzellfunktionsfläche als luminale Besiedelung vorgesehen ist.

7. Implantat nach einem der vorstehenden Ansprüche, ***dadurch gekennzeichnet, dass*** eine umgebende Trennlage vorgesehen ist, insbesondere eine durchlässige Folie, vor allem mikroporös.

8. Implantat nach einem der vorstehenden Ansprüche, ***dadurch gekennzeichnet, dass*** zu einem Lumen (9, 24, 41) des Stents (4, 20. 31) eine Flimmerepitheloberfläche (40) vorgesehen ist.

9. Implantat nach einem der vorstehenden Ansprüche, ***dadurch gekennzeichnet, dass*** in einem Lumen in der tubulären Stützstruktur eine Zusatzstruktur vorgesehen ist, welche eine Ventil- oder Drosselfunktion eines durch die Stützstruktur fließenden biologischen Mediums erlaubt.

10. Implantat nach einem der vorstehenden Ansprüche, ***dadurch gekennzeichnet, dass*** die Stützstruktur zylindrisch geschlossen ausgebildet ist, sodass das Implantat für einen offenen ductus botalli einsetzbar ist.

11. Implantat nach einem der vorstehenden Ansprüche, ***dadurch gekennzeichnet, dass*** die Stützstruktur eine Verankerung zu einer größeren, externen artifiziellen Struktur aufweist oder zu dieser bereits verankert ist, vor allem zu einer Polyurethanstruktur und/oder zu einer künstlichen Ersatzblase und/oder zu einem Kunstherz und/oder zu einem Medikamentenapplikator.

12. Implantat nach Anspruch 11, ***dadurch gekennzeichnet, dass*** die Stützstruktur von der externen Struktur weg verläuft, insbesondere in einem rechten Winkel auf einer Oberfläche der externen Struktur.

13. Implantat nach einem der vorstehenden Ansprüche, ***dadurch gekennzeichnet, dass*** das aktive Gewebe vaskuläre Muskelzellen und Endothelzellen aufweist.

14. Implantat nach einem der vorstehenden Ansprüche, ***dadurch gekennzeichnet, dass*** das aktive Gewebe mehrere Schichten und/oder eine Schicht unterschiedlicher Zellzusammensetzung aufweist.

15. Implantat nach einem der vorstehenden Ansprüche, ***dadurch gekennzeichnet, dass*** die Besiedelung mit dem aktiven Gewebe mittels eines Spritzguss- oder anderen Gussverfahrens, eines dip-coating-Verfahrens, eines Sprühverfahrens oder eines LIFT (laser induced forward transfer)-Verfahrens erzeugt ist.

16. Implantat nach einem der vorstehenden Ansprüche, ***dadurch gekennzeichnet, dass*** an der Stützstruktur für die Besiedelung mit dem aktiven Gewebe eine Trägerstruktur vorgesehen ist, insbesondere ein Hydrogel oder eine synthetische Trägerstruktur.

17. Implantat nach einem der vorstehenden Ansprüche, ***dadurch gekennzeichnet, dass*** das aktive Gewebe dünngestaltig ist, mit einer Schichtdicke zwischen 5 µm und 100 µm, insbesondere zwischen 5 µm und etwa 50 µm, vor allem in der Gesamtbeschichtung auf der Stützstruktur 10 µm bis 20 µm, insbesondere im arithmetischen Mittelwert oder im Median.

18. Implantat nach einem der vorstehenden Ansprüche, ***gekennzeichnet durch*** eine Ausgestaltung als endobronchialer Stent, urologischer Stent, Stent für koronare, periphere Stenosen, gestentete Herzklappenprothese, Okkludersystem für den Verschluss von angeworbenen Defekten (ASD, VSD), Tracheobroncheal-Stent, Stent für Oesophago-Tracheale Fistelverschlüsse, Oesophagus Stent, Darmanastomosierung, Gallengangsstent, Nasennebenhöhlen-Stent, Liquorstent mit integriertem Ventil, Kunstherz-Gefäß, Port-Venensystemstent oder Kunstharnblase-Ureter/Urethra-Stent, insbesondere für die perkutane periphere oder koronare Stentangioplastie.

19. Verfahren zum Herstellen eines Implantats nach einem der vorstehenden Ansprüche, ***dadurch gekennzeichnet, dass*** das aktive Gewebe in vitro mittels eines tissue-engineering-Verfahrens erstellt wird.

20. Verfahren nach Anspruch 19, ***dadurch gekennzeichnet, dass*** zum Besiedeln der Stützstruktur ein Gussverfahren zum Einsatz kommt, insbesondere ein Spritzgussverfahren.

21. Artifizielle Struktur zum Implantieren, insbesondere Polyurethanstruktur und/oder künstliche Ersatzblase und/oder Kunstherz und/oder Medikamentenapplikator, im Kit zum Verankern mit einem Implantat nach einem der Ansprüche 1 bis 18.

22. Artifizielle Struktur zum Implantieren, insbesondere Polyurethanstruktur und/oder künstliche Ersatzblase und/oder Kunstherz und/oder Medikamentenapplikator, verankert mit einem Implantat nach einem der Ansprüche 1 bis 18.

23. Verwendung eines Implantats nach einem der Ansprüche 1 bis 18 als endobronchialer Stent, urologischer Stent, Stent für koronare, periphere Stenosen, gestentete Herzklappenprothese, Okkludersystem für den Verschluss von angeworbenen Defekten (ASD, VSD), Tracheobroncheal-Stent, Stent für Oesophago-Tracheale Fistelverschlüsse, Oesophagus Stent, Darmanastomosierung, Gallengangsstent, Nasennebenhöhlen-Stent, Liquorstent mit integriertem Ventil, Kunstherz-Gefäß, Port-Venensystemstent oder Kunstharnblase-Ureter/Urethra-Stent, insbesondere für die perkutane periphere oder koronare Stentangioplastie.

## Claims

1. An implant having a mechanical component and a biologically active component, wherein the mechanical component comprises a shaping support structure (6, 22, 34) and the biologically active component comprises a biocompatible material with cells to colonize the support structure (6, 22, 34), ***characterized in that*** the cells form a living, vascular active tissue (7) and the support structure (6, 22, 34) is completely integrated into the active tissue (7);
wherein the implant is constructed as a stent (4, 20, 31) having a tubular support structure (6, 22, 34) which has a radial stiffness in order to act as a support for a vessel (1);
wherein the active tissue (7) forms a hollow cylinder and the tubular support structure (6, 22, 34) is integrated into a cylinder wall;
wherein the tubular support structure (6, 22, 34) is completely integrated into living, biologically active tissue (7), whereupon complete coating can be obtained; and
wherein the tissue defined as "active" comprises living cells which can contribute to obtaining the implant function by means of the production and release of factors with or without prior stimulation or by their physiological localization.

2. The implant as claimed in claim 1, ***characterized in that*** it consists exclusively of the two components.

3. The implant as claimed in claim 1 or claim 2, ***characterized in that*** the stent (4, 20, 31) is radially compressible and/or expandable, in particular balloon expandable or self-expandable.

4. The implant as claimed in one of the preceding claims, ***characterized in that*** the stent (4, 20, 31) is constructed so as to seal to the tubular support structure (6, 22, 34) so that, following implantation of the stent (4, 20, 31), a damaged wall section of a vessel (1), in particular an artherosclerotic wall section, is completely cut off from a flow of blood.

5. The implant as claimed in one of the preceding claims, ***characterized in that*** the active tissue (7) comprises a functional endothelial cell surface, in particular an endothelial cell surface with functionally active endothelial cells.

6. The implant as claimed in claim 5, ***characterized in that*** the functional endothelial cell surface is provided as luminal colonization.

7. The implant as claimed in one of the preceding claims, ***characterized in that*** a surrounding separating layer is provided, in particular a permeable film, in particular microporous.

8. The implant as claimed in one of the preceding claims, ***characterized in that*** one lumen (9, 24, 41) of the stent (4, 20, 31) is provided with a ciliated endothelial surface (40).

9. The implant as claimed in one of the preceding claims, ***characterized in that*** an additional structure is provided in a lumen in the tubular support structure, which provides a valve or throttle function for a biological medium flowing through the support structure.

10. The implant as claimed in one of the preceding claims, ***characterized in that*** the support structure is formed as a closed cylinder so that the implant can be used for a patent ductus arteriosus.

11. The implant as claimed in one of the preceding claims, ***characterized in that*** the support structure comprises an anchor for a larger, external artificial structure or is already anchored thereto, in particular to a polyurethane structure and/or to an artificial replacement bladder and/or to an artificial heart and/or to an applicator for medication.

12. The implant as claimed in claim 11, ***characterized in that*** the support structure extends outwardly from the outer structure, in particular at a right angle to a surface of the outer structure.

13. The implant as claimed in one of the preceding claims, ***characterized in that*** the active tissue comprises vascular muscle cells and endothelial cells.

14. The implant as claimed in one of the preceding claims, ***characterized in that*** the active tissue comprises a plurality of layers and/or a layer with a differing cell composition.

15. The implant as claimed in one of the preceding claims, ***characterized in that*** the colonization with the active tissue is produced by means of an injection moulding or other moulding process, a dip coating process, a spray process or a LIFT (laser induced forward transfer) process.

16. The implant as claimed in one of the preceding claims, ***characterized in that*** a carrier structure is provided on the support structure for colonization with the active tissue, in particular a hydrogel or a synthetic carrier structure.

17. The implant as claimed in one of the preceding claims, ***characterized in that*** the active tissue is thin in shape, with a layer thickness in the range 5 µm to 100 µm, in particular in the range 5 µm to 50 µm, especially a total coating on the support structure of 10 µm to 20 µm, in particular as the arithmetic mean or median.

18. The implant as claimed in one of the preceding claims, ***characterized in that*** it is formed as an endobronchial stent, a urological stent, a stent for coronary, periphery stenoses, a stented heart valve prosthesis, an occluding system for closing acquired defects (ASD, VSD), a tracheobronchial stent, a stent for closing oesophago-tracheal fistulas, an oesophageal stent, an intestinal anastomosis stent, a bile duct stent, a paranasal sinus stent, a cerebrospinal fluid stent with integrated valve, an artificial heart vessel, a portal vascular system stent or artificial bladder ureter/urethra stent, in particular for percutaneous, peripheral or coronary stent angioplasty.

19. A method for manufacturing an implant as claimed in one of the preceding claims, ***characterized in that*** the active tissue is produced in vitro by means of a tissue engineering process.

20. The method as claimed in claim 19, ***characterized in that*** an injection process, in particular an injection moulding process, is used to colonize the support structure.

21. An artificial structure for implantation, in particular a polyurethane structure and/or an artificial replacement bladder and/or artificial heart and/or applicator for medication, as a kit for anchoring with an implant as claimed in one of claims 1 to 18.

22. An artificial structure for implantation, in particular a polyurethane structure and/or an artificial replacement bladder and/or artificial heart and/or applicator for medication, anchored with an implant as claimed in one of claims 1 to 18.

23. Use of an implant as claimed in one of claims 1 to 18 as an endobronchial stent, a urological stent, a stent for coronary, periphery stenoses, a stented heart valve prosthesis, an occluding system for closing acquired defects (ASD, VSD), a tracheobronchial stent, a stent for closing oesophago-tracheal fistulas, an oesophageal stent, an intestinal anastomosis stent, a bile duct stent, a paranasal sinus stent, a cerebrospinal fluid stent with integrated valve, an artificial heart vessel, a portal vascular system stent or artificial bladder ureter/urethra stent, in particular for percutaneous, peripheral or coronary stent angioplasty.

## Revendications

1. Implant comprenant une composante mécanique et une composante active biologiquement, sachant que la composante mécanique présente une structure de soutien (6, 22, 34) formatrice et la composante active biologiquement présente un matériau avec des cellules, biocompatible et colonisant la structure de soutien (6, 22, 34), ***caractérisé en ce que*** les cellules forment un tissu (7) vital actif sur le plan vasculaire, et la structure de soutien (6, 22, 34) est totalement intégrée dans le tissu actif (7),
sachant que l'implant est conçu en tant que stent (4, 20, 31), comprenant une structure de soutien (6, 22, 34) tubulaire qui présente une rigidité radiale pour une action de soutien d'un vaisseau (1),
sachant que le tissu actif (7) forme un cylindre creux et la structure de soutien (6, 22, 34) tubulaire est intégrée dans une paroi cylindrique,
sachant qu'une intégration complète de la structure de soutien (6, 22, 34) tubulaire dans du tissu vivant, actif biologiquement existe, ce qui permet d'obtenir un revêtement complet, et
sachant que le tissu désigné comme « actif » comprend des cellules vivantes qui peuvent participer, par la production et la libération de facteurs avec ou sans stimulation préalable ou par leur localisation physiologique, à l'obtention du fonctionnement de l'implant.

2. Implant selon la revendication 1, *caractérisé en ce qu*'il est composé exclusivement des deux composantes.

3. Implant selon la revendication 1 ou 2, ***caractérisé en ce que*** le stent (4, 20, 31) est conçu en pouvant subir une compression et/ou une expansion radiale, en particulier une expansion par ballon ou une auto-expansion.

4. Implant selon l'une des revendications précédentes, ***caractérisé en ce que*** le stent (4, 20, 31) est conçu en étanchéité sur la structure de soutien (6, 22, 34) tubulaire, de telle sorte qu'un tronçon de paroi endommagé d'un vaisseau (1), en particulier un tronçon de paroi arthérosclérotique peut être exclu totalement d'une trajectoire du flux sanguin après implantation du stent (4, 20, 31).

5. Implant selon l'une des revendications précédentes, ***caractérisé en ce que*** le tissu actif (7) présente une surface fonctionnelle des cellules endothéliales, en particulier une surface de cellules endothéliales comprenant des cellules endothéliales actives sur le plan fonctionnel.

6. Implant selon la revendication 5, ***caractérisé en ce que*** la surface fonctionnelle des cellules endothéliales est prévue en tant que colonisation luminale.

7. Implant selon l'une des revendications précédentes, ***caractérisé en ce qu'**une* couche de séparation périphérique est prévue, en particulier un film perméable, avant tout microporeux.

8. Implant selon l'une des revendications précédentes, ***caractérisé en ce qu'**une* surface épithéliale vibratile (40) est prévue vers une lumière (9, 24, 41) du stent (4, 20, 31).

9. Implant selon l'une des revendications précédentes, ***caractérisé en ce qu'**une* structure supplémentaire est prévue dans une lumière dans la structure de soutien tubulaire, laquelle permet une fonction de clapet ou d'étranglement pour un agent biologique s'écoulant à travers la structure de soutien.

10. Implant selon l'une des revendications précédentes, ***caractérisé en ce que*** la structure de soutien est fermée cylindriquement, de telle sorte que l'implant peut être utilisé pour un ductus botalli ouvert.

11. Implant selon l'une des revendications précédentes, ***caractérisé en ce que*** la structure de soutien présente un ancrage à une structure artificielle externe plus grande ou est déjà ancrée à celle-ci, avant tout à une structure en polyuréthane et/ou une vessie artificielle et/ou un coeur artificiel et/ou un applicateur de médicament.

12. Implant selon la revendication 11, ***caractérisé en ce que*** la structure supplémentaire s'éloigne de la structure externe, en particulier en angle droit sur une surface de la structure externe.

13. Implant selon l'une des revendications précédentes, ***caractérisé en ce que*** le tissu actif présente des cellules musculaires et des cellules endothéliales vasculaires.

14. Implant selon l'une des revendications précédentes, ***caractérisé en ce que*** le tissu actif présente plusieurs couches et/ou une couche de composition cellulaire différente.

15. Implant selon l'une des revendications précédentes, ***caractérisé en ce que*** la colonisation avec le tissu actif est produite par un procédé de moulage par injection ou autre procédé de moulage, un procédé de revêtement par immersion, un procédé de pulvérisation ou un procédé LIFT (laser induced forward transfer).

16. Implant selon l'une des revendications précédentes, ***caractérisé en ce qu'**une* structure porteuse est prévue sur la structure de soutien pour la colonisation avec le tissu actif, en particulier un hydrogel ou une structure porteuse synthétique.

17. Implant selon l'une des revendications précédentes, ***caractérisé en ce que*** le tissu actif est fin, avec une épaisseur de couche entre 5 µm et 100 µm, en particulier entre 5 µm et 50 µm, avant tout de 10 µm à 20 µm dans la totalité du revêtement sur la structure de soutien, en particulier dans la valeur moyenne arithmétique ou dans la médiane.

18. Implant selon l'une des revendications précédentes, ***caractérisé* par** une conception en tant que stent endobronchial, stent urologique, stent pour sténoses coronaires périphériques, prothèse de valvule avec stent, système d'obturateur pour la fermeture de défauts engagés (communications interauriculaires - ASD, communications interventriculaires - VSD), stent trachéobronchial, stent pour fermeture de fistules oeso-trachéales, stent de l'oesophage, anastomose de l'intestin, stent des voies biliaires, stent des sinus, stent de liquide avec clapet intégré, vaisseau de coeur artificiel, stent de système veineux porte ou stent pour vessie artificielle-uretère/urètre, en particulier pour l'angioplastie par stent périphérique ou coronaire percutanée.

19. Procédé de production d'un implant selon l'une des revendications précédentes, ***caractérisé en ce que*** le tissu actif est fabriqué in vitro via un procédé d'ingénierie des tissus.

20. Procédé selon la revendication 19, ***caractérisé en ce que*** pour coloniser la structure de soutien, on emploie un procédé de moulage, en particulier un procédé de moulage par injection.

21. Structure artificielle destinée à être implantée, en particulier structure en polyuréthane et/ou vessie artificielle et/ou coeur artificiel et/ou applicateur de médicament, en kit pour ancrage avec un implant selon l'une des revendications 1 à 18.

22. Structure artificielle destinée à être implantée, en particulier structure en polyuréthane et/ou vessie artificielle et/ou coeur artificiel et/ou applicateur de médicament, en kit, ancrée avec un implant selon l'une des revendications 1 à 18.

23. Emploi d'un implant selon l'une des revendications 1 à 18, en tant que stent endobronchial, stent urologique, stent pour sténoses coronaires périphériques, prothèse de valvule avec stent, système d'obturateur pour la fermeture de défauts engagés (communications interauriculaires - ASD, communications interventriculaires - VSD), stent trachéobronchial, stent pour fermeture de fistules oeso-trachéales, stent de l'oesophage, anastomose de l'intestin, stent des voies biliaires, stent des sinus, stent de liquide avec clapet intégré, vaisseau de coeur artificiel, stent de système veineux porte ou stent pour vessie artificielle-uretère/urètre, en particulier pour l'angioplastie par stent périphérique ou coronaire percutanée.
